# EUROPÄISCHE PATENTSCHRIFT

(11) **EP 2 812 072 B1**
(45) Veröffentlichungstag und Bekanntmachung des Hinweises auf die Patenterteilung: **28.09.2016**
(21) Anmeldenummer: 13704001.0
(22) Anmeldetag: 06.02.2013
(51) Int. Cl.: A61N 5/10

(54) **ABWINKELBARER APPLIKATOR FÜR STRAHLENTHERAPIE**
FLEXIBLE APPLICATOR FOR RADIATION THERAPY
APPLICATEUR POUVANT ÊTRE COUDÉ, POUR LA RADIOTHÉRAPIE

(30) Priorität: 08.02.2012 DE 102012002466
(43) Veröffentlichungstag der Anmeldung: 17.12.2014
(73) Patentinhaber: Carl Zeiss Meditec AG, 07745 Jena (DE)
(72) Erfinder: KLEINWAECHTER, Timo, 73430 Aalen (DE); HAUSAM, Volker, 78224 Singen (DE); REITER, Zoran, 78576 Emmingen (DE)
(74) Vertreter: Carl Zeiss AG - Patentabteilung
(86) Internationale Anmeldenummer: PCT/EP2013/000349
(87) Internationale Veröffentlichungsnummer: WO 2013/117324

(56) Entgegenhaltungen:
- WO-A1-2006/031771
- DE-C1- 4 413 491
- US-A- 5 036 201

## Beschreibung

Die vorliegende Erfindung betrifft einen Applikator zur Anwendung bei der Strahlentherapie, umfassend einen Grundkörper zur Aufnahme einer Strahlungsquelle in einem Hohlraum des Grundkörpers und einen Absorptionskörper, welcher eine Öffnung aufweist, durch welche Strahlung austreten kann. Die Größe und die Form der Austrittsöfnung des Absorptionskörpers bestimmt dabei die Austrittsrichtung und den Querschnitt der austretenden Strahlung.

### Technischer Hintergrund

Bei der intraoperativen Strahlentherapie (IORT) ist es oft notwendig, Gewebe zu bestrahlen, welches nicht einfach zugänglich ist. Bei bekannten Applikatoren zur Bestrahlung einer Oberfläche kann nur Gewebe bestrahlt werden, welches in Richtung einer Hauptachse des Applikators von außen erreichbar ist. Dies führt dazu, dass beispielsweise bei einer Bestrahlung des Beckenknochens während des Eingriffs ein Großteil des Darms zur Seite bewegt werden muss, um Zugang zum zu bestrahlenden Gewebe zu bekommen. Ein gewinkelter Applikator könnte seitlich am Darm vorbei geführt werden und die gewünschte Fläche am Beckenknochen bestrahlen. Für unterschiedliche Anwendungen ist hierbei jedoch jeweils ein unterschiedlicher Winkel des Applikators optimal.

Bei manchen Bestrahlungsgeräten wird für die intraoperative Strahlentherapie eine Strahlungsquelle verwendet, welche gleichförmig in alle Richtungen strahlt. Zum Erreichen gewünschter Strahlungscharakteristiken ist es jedoch üblich, einige Raumrichtungen der emittierten Strahlung selektiv zu absorbieren, so dass eine bevorzugte Strahlungsrichtung definiert wird.

Aus WO 2006/031771 A1 ist ein Applikator zur Anwendung bei der Strahlentherapie bekannt, bei dem ein zylinderförmiger Endbereich eines Applikators gegenüber einem Applikatorschaft abgewinkelt ist.

Es ist Aufgabe der vorliegenden Erfindung, einen Applikator für die Strahlentherapie bereitzustellen, bei welchem die bevorzugte Strahlungsrichtung relativ zu einer Hauptachse des Applikators verstellbar ist, und welcher einfach zu reinigen ist.

Diese Aufgabe wird durch einen Applikator gemäß Anspruch 1 gelöst. Weitere vorteilhafte Ausgestaltungen sind in den Unteransprüchen angegeben.

Ein erfindungsgemäßer Applikator zur Anwendung bei der Strahlentherapie umfasst:
- einen Grundkörper zur Aufnahme einer Strahlungsquelle, wobei der Grundkörper einen Hohlraum in seinem inneren aufweist, welcher sich entlang einer Grundkörper-Hauptachse erstreckt; und
- einen Absorptionskörper, welcher mit dem Grundkörper bewegbar verbunden ist, und welcher die aus der Strahlungsquelle austretende Strahlung derart beeinflusst, dass eine bevorzugte Strahlungsrichtung definiert ist,
wobei der Absorptionskörper gegenüber dem Grundkörper derart bewegbar ist, dass die bevorzugte Strahlungsrichtung relativ zur Grundkörper-Hauptachse in eine Mehrzahl unterschiedlicher Stellungen verkippt werden kann.

Ein derartiger Applikator ermöglicht die Bestrahlung von Gewebe, welches für herkömmliche Applikatoren nicht einfach zugänglich ist, da es nicht in gerader Linie von außen erreichbar ist. Bei dem erfindungsgemäßen Applikator kann die Austrittsrichtung der Strahlung relativ zur Hauptachse des Applikator-Grundkörpers derart verkippt werden, dass auch Gewebe erreichbar ist, welches nicht in normal zu einer Hauptachse des Applikators liegt.

Vorzugsweise ist der Absorptionskörper mit dem Grundkörper lösbar verbunden, um eine einfache Reinigung und Sterilisierung des Applikators zu ermöglichen.

Gemäß einer Ausführungsform des erfindungsgemäßen Applikators ist der Absorptionskörper mit dem Grundkörper drehbar verbunden, wobei die Drehachse des Absorptionskörpers gegenüber der Grundkörper-Hauptachse geneigt ist. Hierdurch kann die Austrittsrichtung der Strahlung durch eine Drehung des Absorptionskörpers relativ zum Grundkörper geändert werden.

Weiterhin kann ein Arretierungsmittel vorgesehen sein, um die Stellung des Absorptionskörpers relativ zum Grundkörper zu arretieren. Gemäß einer Ausführungsform der vorliegenden Erfindung umfasst das Arretierungsmittel einen Sicherungsring, welcher auf konstruktiv einfache Art und Weise eine zuverlässige Arretierung des Absorptionskörpers ermöglicht.

Der Absorptionskörper kann dabei in einer Mehrzahl von unterschiedlichen Stellungen relativ zum Grundkörper arretierbar sein, wobei die Austrittsrichtung der Strahlung relativ zur Grundkörper-Hauptachse für jede Stellung einen anderen Wert annimmt. Somit wird ein Applikator bereitgestellt, bei welchem die Austrittsrichtung der Strahlung in eine Mehrzahl von unterschiedlichen Richtungen relativ zur Grundkörper-Hauptachse eingestellt und dort jeweils arretiert werden kann.

Gemäß einer anderen Ausführungsform kann der Absorptionskörper stufenlos relativ zum Grundkörper bewegbar sein und in jeder beliebigen Stellung durch das Arretierungsmittel arretierbar sein. Hierdurch kann, innerhalb eines vorgegebenen Bewegungsbereichs des Absorptionskörpers, jeder beliebige Winkel der Austrittsrichtung der Strahlung relativ zur Richtung der Grundköper-Hauptachse eingestellt werden.

Der Absorptionskörper kann einen Absorptionsmittelhalter und ein Absorptionsmittel umfassen, wobei das Absorptionsmittel derart ausgebildet ist, dass es Strahlcharakteristiken, wie beispielsweise Durchmesser, Intensität und Ausbreitungsrichtung der austretenden Strahlung beeinflusst. Das Absorptionsmittel kann beispielsweise als Blende ausgestaltet sein, um einen Durchmesser der austretenden Strahlung zu definieren, oder das Absorptionsmittel kann Strahlcharakteristiken, wie beispielsweise die Eindringtiefe in das zu bestrahlende Gewebe, definieren.

Das Absorptionsmittel kann hierbei jedes Bauteil umfassen, welches beispielsweise aufgrund der räumlichen Verteilung seiner Dichte und/oder seiner Materialstärke die von der Strahlungsquelle erzeugte Strahlung beeinflussen kann.

Dieser zweiteilige Aufbau des Absorptionskörpers ist vorteilhaft, da Form und Material des Absorptionsmittelhalters im Hinblick auf die Bewegung und Arretierung des Absorptionskörpers optimiert werden können, und unabhängig von diesen Überlegungen die gewünschten Absorptionscharakteristiken für die verwendete Strahlung durch geeignete Auswahl des Absorptionsmittels realisiert werden können.

Weiterhin umfasst die vorliegende Erfindung ein Applikatorsystem zur Anwendung bei der Strahlentherapie, umfassend den voranstehend beschriebenen Applikator und eine Mehrzahl von Absorptionskörpern, welche jeweils mit dem Grundkörper des Applikators verbindbar sind und die aus der Strahlungsquelle austretende Strahlung jeweils derart beeinflussen, dass eine bevorzugte Strahlungsrichtung und jeweils unterschiedliche Strahlcharakteristiken der austretenden Strahlung definiert sind. Ein derartiges Applikatorsystem ermöglicht somit nicht nur die Bestrahlung von Gewebe mit unterschiedlichen Winkeln relativ zur Hauptachse des Applikators, sondern auch eine einfache Einstellung unterschiedlicher gewünschter Strahlungsquerschnitte und Strahlungscharakteristiken.

Im folgenden werden einige Ausführungsformen der vorliegenden Erfindung beispielhaft anhand der beiliegenden Zeichnungen erläutert. Es zeigen:
- Figur 1: eine Ansicht eines Applikators gemäß einer Ausführungsform der vorliegenden Erfindung;
- Figur 2a - 2d: unterschiedliche Stellungen des Absorptionskörpers des in Figur 1 gezeigten Applikators;
- Figur 3: den Grundkörper, einen Sicherungsring und einen dazugehörigen Federring;
- Figur 4: einen Querschnitt durch den in Figur 2d gezeigten Applikator;
- Figur 5: eine Detailansicht des in Figur 4 gezeigten Querschnitts; und
- Figur 6a - 6c: unterschiedliche Stellungen des Sicherungsrings am Applikator-Grundkörper.

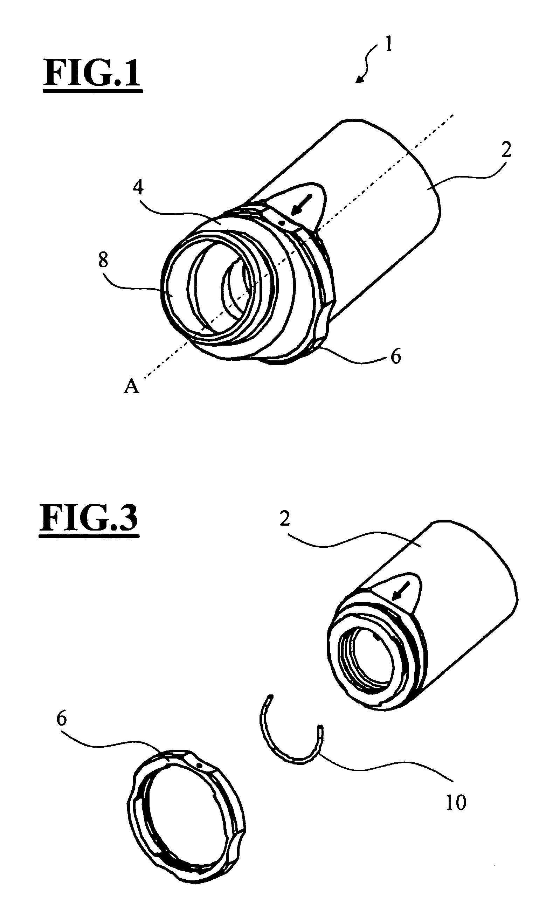

Wie in Figur 1 gezeigt, umfasst ein erfindungsgemäßer Applikator 1 einen Grundkörper 2, einen drehbar am Grundkörper 2 befestigten Absorptionskörper 4 und einen Sicherungsring 6.

Der zylinderförmige Grundkörper 2 weist in seinem Inneren einen Hohlraum auf, in den eine Strahlungsquelle (nicht gezeigt) eingeführt werden kann. Die Strahlungsquelle kann hierbei jede Strahlungsquelle umfassen, die sich für einen Einsatz in der intraoperativen Strahlungstherapie eignet. Bei einigen Bestrahlungsgeräten für die IORT wird die Strahlenquelle in einem Endbereich des Applikators 1 angebracht, so dass die Strahlenquelle während der Therapie möglichst nah an das zu bestrahlende Gewebe gebracht werden kann.

Der Absorptionskörper 4 ist aus einem Material gebildet, welches die von der Strahlungsquelle austretende Strahlung absorbiert. Form und Material des Absorptionskörpers 4 sind hierbei so gestaltet, dass die aus der Strahlungsquelle kugelförmig austretende Strahlung selektiv absorbiert wird, so dass im Wesentlichen nur Strahlung in einer bevorzugten Strahlungsrichtung aus dem Absorptionskörper 4 austritt. Hierzu kann eine beispielhafte Ausführungsform eines Absorptionskörpers 4 an seinem Ende eine Austrittsöfnung 8 aufweisen, durch welche die Strahlung austreten kann. Die Richtung der austretenden Strahlung relativ zu einer Hauptachse A des Grundkörpers 2 wird in diesem Fall durch die Lage und Ausrichtung der Austrittsöfnung 8 relativ zur Hauptachse A des Grundkörpers 2 definiert.

In die Austrittsöfnung 8 kann ein Absorptionsmittel (nicht gezeigt) eingesetzt werden, welches derart ausgestaltet ist, dass die Strahlcharakteristiken der austretenden Strahlung entsprechend der gewünschten Anwendung eingestellt werden können, beispielsweise entsprechend der gewünschten Eindringtiefe in das zu bestrahlende Gewebe. Unter einem Absorptionsmittel ist in diesem Zusammenhang jedes Bauteil zu verstehen, welches zur Beeinflussung von Strahlcharakteristiken wie beispielsweise der Strahlform und -stärke geeignet ist. Alternativ ist auch ein Absorptionsmittel in Form einer Blende denkbar, oder es ist denkbar, die aus der Austrittsöfnung 8 austretende Strahlung ohne weitere Beeinflussung zur Bestrahlung des Gewebes zu verwenden.

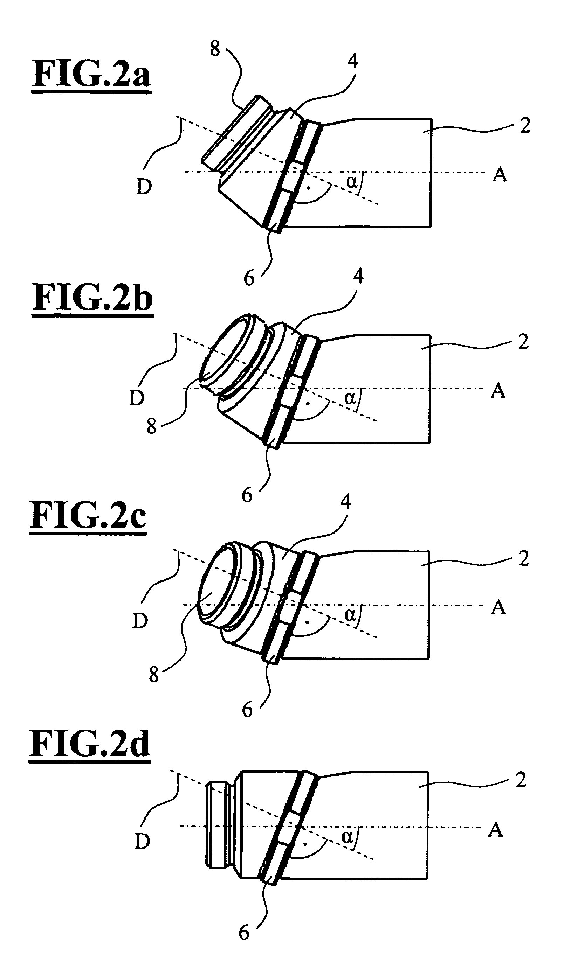

Wie in Figuren 2a bis 2d gezeigt, ist der Absorptionskörper 4 um eine Drehachse D drehbar, welche um einen Winkel α zur Hauptachse A des Grundkörpers 2 verkippt ist. Bei der gezeigten Ausführungsform beträgt der Winkel α 20°, so dass eine maximale Abwinklung der Austrittsrichtung der Strahlung gegenüber der Hauptachse A des Grundkörpers 2 um 40° erreicht wird (siehe Fig. 2a). Es sind auch andere Werte für α, und somit für den maximalen Wert der Abwinklung der Austrittsrichtung der Strahlung gegenüber der Grundkörper-Hauptachse A denkbar.

Wie aus Fig. 2d ersichtlich, ist bei der vorliegenden Erfindung die Drehung des Absorptionskörpers 4 um eine relativ zur Hauptachse A des Grundkörpers 2 gekippte Drehachse D konstruktiv besonders einfach dadurch verwirklicht, dass Grundkörper 2 und Absorptionskörper 4 jeweils im Wesentlichen zylinderförmig ausgebildet sind, wobei die beiden Zylinder in Längsrichtung hintereinander angeordnet sind. Die Ebene, in welcher die Enden der beiden Zylinder aneinander stoßen, ist relativ zur Hauptachse A des Grundkörpers 2 geneigt. Dadurch kann auf einfache Weise ein Drehgelenk realisiert werden, welches eine Verschwenkung der Austrittsöfnung 8 der Strahlung gegenüber der Grundkörper-Hauptachse A ermöglicht und welches lediglich einen, einfach zu reinigenden und gut zugänglichen Spalt zwischen dem Grundkörper 2 und dem Absorptionskörper 4 aufweist, in den bei der Benutzung Blut oder Verschmutzungen eindringen können.

Wie in den Figuren 2a bis 2d gezeigt, bewirkt eine Drehung des Absorptionskörpers 4 relativ zum Grundkörper 2 eine Änderung des Winkels der Austrittsrichtung der Strahlung gegenüber der Grundkörper-Hauptachse A. In Figuren 2a und 2d sind dabei die maximal und die minimal möglichen Werte für den Bewegungsbereich der Austrittsrichtung der Strahlung gegenüber der Grundkörper-Hauptachse A gezeigt.

Wie aus Figur 3 ersichtlich, sind zur Befestigung des Absorptionskörpers 4 am Grundkörper 2 der Sicherungsring 6 und ein Federring 10 vorgesehen.

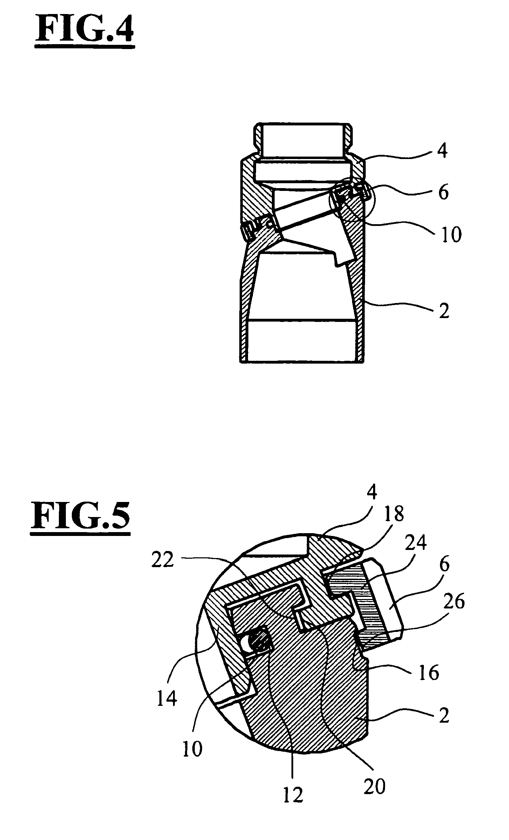

Das Zusammenwirken von Sicherungsring 6 und Federring 10 ist in Figuren 4 und 5 dargestellt. Der Federring 10 liegt in einer Nut 12 in der Innenfläche des Grundkörpers 2. Eine Anlagefläche 14 des Absorptionskörpers 4 ist derart in Kontakt mit dem Federring 10, dass die Reibung zwischen dem Federring 10 und der Anlagefläche 14 eine ungewollte Rotation des Absorptionskörpers 4 relativ zum Grundkörper 2 verhindert.

Der Sicherungsring 6 greift mit entsprechenden Auskragungen 24, 26 in Nuten 16, 18 an den jeweiligen Außenflächen des Grundkörpers 2 und des Absorptionskörpers 4 ein. Als zusätzliche axiale Sicherung des Absorptionskörpers 4 relativ zum Grundkörper 2 greift weiterhin ein Vorsprung 20 des Absorptionskörpers 4 in eine Nut 22 an der Außenfläche des Grundkörpers 2 ein.

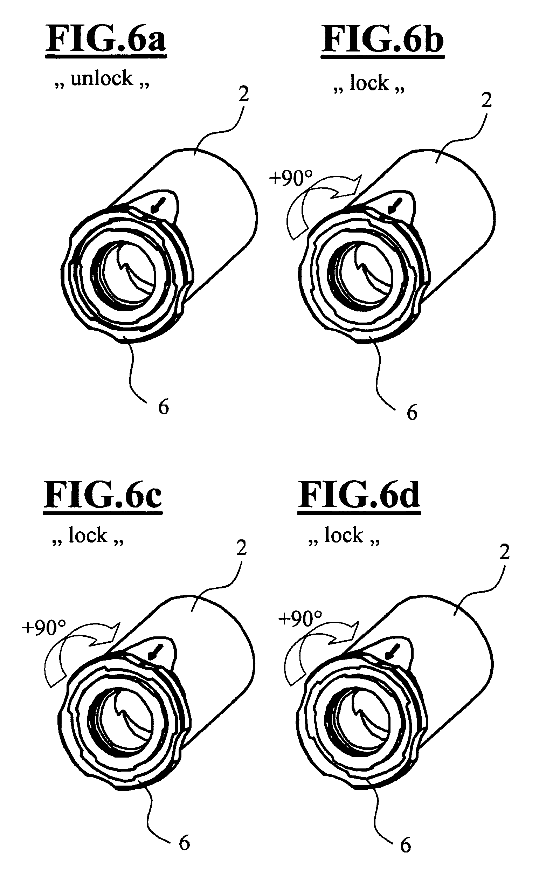

Dabei sind die entsprechenden Nuten und Vorsprünge jeweils nicht auf dem gesamten Umfang des Grundkörpers 2, des Sicherungsrings 6 und des Absorptionskörpers 4 vorgesehen. Verdickungen und Freimachungen der Nut 16 des Grundkörpers 2 und des Auskragung 26 des Sicherungsrings 6 wechseln sich derart ab, dass durch eine Drehung des Sicherungsrings 6, wie in Figuren 6b bis 6c gezeigt, der Sicherungsring 6 in den mit "lock" gekennzeichneten Stellungen den Absorptionskörper 4 am Grundkörper 2 axial sichert, während eine Drehung des Absorptionskörpers 4 relativ zum Grundkörper 2 weiterhin möglich ist.

In der in Fig. 6a gezeigten und mit "unlock" gekennzeichneten Freigabestellung des Sicherungsrings 6 kann der Absorptionskörper 4 in axialer Richtung vom Grundkörper 2 abgenommen werden. Dazu muss allerdings der Absorptionskörper 4 derart relativ zum Grundkörper 2 verdreht werden, dass auch der Eingriff zwischen den jeweiligen Nuten und Vorsprüngen des Absorptionskörpers 4 und des Grundkörpers 2 aufgehoben wird.

Somit ermöglicht der Sicherungsring 6 die einfache Montage und Demontage des Absorptionskörpers 4 relativ zum Grundkörper 2. Dies ist insbesondere im Hinblick auf die Reinigung des Drehgelenks zwischen Grundkörper 2 und Absorptionskörper 4 vorteilhaft. Gemäß einer Weiterbildung der vorliegenden Ausführungsform können unterschiedliche Absorptionskörper 4 bereitgestellt werden, wobei die Austrittsöffnungen jeweils unterschiedlich bemessen und ausgestaltet sind, um jeweils unterschiedliche Strahlcharakteristiken der austretenden Strahlung bereitzustellen. Ein Benutzer kann dann für jede Anwendung den passenden Absorptionskörper 4 montieren, ohne jeweils den kompletten Applikator 1 austauschen zu müssen.

Durch eine entsprechende Ausgestaltung der Nut 18 am Absorptionskörper 4 und der Auskragung 24 am Sicherungsring 6 ist es weiterhin möglich, den Sicherungsring 6 für eine Reinigung vom Absorptionskörper 4 zu lösen.

Der Eingriff zwischen dem Grundkörper 2 und dem Sicherungsring 6 kann hierbei dermaßen ausgestaltet sein, dass sich der Sicherungsring 6 jeweils rastend um 90° gegenüber dem Grundkörper verdrehen lässt, so dass der Benutzer die jeweiligen Arretier- und Freigabestellungen des Sicherungsrings 6 einfach einstellen kann. Hierzu sind beispielsweise zwei Aufdickungen in der Schnappnut 16 des Grundkörpers 2 und vier Freimachungen in der Auskragung 26 des Sicherungsrings 6 vorgesehen.

Der in der vorliegenden Ausführungsform gezeigte Applikator 1 ermöglicht eine stufenlose Verstellung des Winkels der Austrittsöfnung 8 gegenüber der Grundkörper-Hauptachse A in einem Winkelbereich zwischen 0 und 40°. Es ist jedoch auch denkbar, beispielsweise mittels eines geeignet ausgestalteten Rastmechanismus, eine Mehrzahl von diskreten Stellungen des Absorptionskörpers 4 relativ zum Grundkörper 2 vorzusehen. Ein derartiger Verstellmechanismus mit einer Mehrzahl von definierten Stellungen des Absorptionskörpers 4 relativ zum Grundkörper 2 ermöglicht es einem Benutzer, eine Anzahl von bestimmten, vorgegebenen Winkeln jeweils genau einzustellen.

### Bezugszeichenliste

- 1: Applikator
- 2: Grundkörper
- 4: Absorptionskörper
- 6: Sicherungsring
- 8: Austrittsöfnung
- 10: Federring
- 12: Nut
- 14: Anlagefläche
- 16: Nut
- 18: Nut
- 20: Vorsprung
- 22: Nut
- 24: Auskragung
- 26: Auskragung

- A: Hauptachse des Grundkörpers
- D: Drehachse des Absorptionskörpers
- α: Winkel zwischen A und D

## Patentansprüche

1. Applikator (1) zur Anwendung bei der Strahlentherapie, umfassend:
- einen im Wesentlichen zylinderförmig ausgebildeten Grundkörper (2) zur Aufnahme einer Strahlungsquelle, wobei der Grundkörper (2) einen Hohlraum in seinem Inneren aufweist, welcher sich entlang einer Grundkörper-Hauptachse (A) erstreckt; und
- einen im Wesentlichen zylinderförmig ausgebildeten Absorptionskörper (4), welcher mit dem Grundkörper (2) bewegbar verbunden ist, und welcher die aus der Strahlungsquelle austretende Strahlung derart beeinflusst, dass eine bevorzugte Strahlungsrichtung definiert ist,
wobei der Grundkörper (2) und der Absorptionskörper (4) in Längsrichtung hintereinander angeordnet sind und die Ebene, in welcher die Enden der beiden Zylinder aneinander stoßen, relativ zur Grundkörper-Hauptachse (A) geneigt ist,
**dadurch gekennzeichnet, dass** der Absorptionskörper (4) mit dem Grundkörper (2) drehbar verbunden ist und die Drehachse (D) des Absorptionskörpers (4) gegenüber der Grundkörper-Hauptachse (A) geneigt ist, so dass die bevorzugte Strahlungsrichtung relativ zur Grundkörper-Hauptachse (A) in eine Mehrzahl unterschiedlicher Stellungen verkippt werden kann.

2. Applikator (1) nach Anspruch 1, **dadurch gekennzeichnet, dass** der Absorptionskörper (4) mit dem Grundkörper (2) lösbar verbunden ist.

3. Applikator (1) nach einem der Ansprüche 1 oder 2, **dadurch gekennzeichnet, dass** ein Arretierungsmittel (6) vorgesehen ist, um eine Stellung des Absorptionskörpers (4) gegenüber dem Grundkörper (2) zu arretieren.

4. Applikator (1) nach Anspruch 3, **dadurch gekennzeichnet, dass** das Arretierungsmittel einen Sicherungsring (6) umfasst.

5. Applikator (1) nach einem der Ansprüche 3 oder 4, **dadurch gekennzeichnet, dass** der Absorptionskörper (4) in einer Mehrzahl von unterschiedlichen Stellungen relativ zum Grundkörper (2) arretierbar ist, wobei die bevorzugte Strahlungsrichtung relativ zur Grundkörper-Hauptachse (A) für jede Stellung einen anderen Wert annimmt.

6. Applikator (1) nach einem der Ansprüche 3 oder 4, **dadurch gekennzeichnet, dass** der Absorptionskörper (4) stufenlos relativ zum Grundkörper (2) bewegbar ist und in jeder beliebigen Stellung durch das Arretierungsmittel (6) arretierbar ist.

7. Applikator (1) nach einem der Ansprüche 1 bis 6, **dadurch gekennzeichnet, dass** der Absorptionskörper (4) einen Absorptionsmittelhalter und ein Absorptionsmittel umfasst, wobei das Absorptionsmittel derart ausgebildet ist, dass es Strahlcharakteristiken, wie beispielsweise Durchmesser, Intensität und Ausbreitungsrichtung der austretenden Strahlung beeinflusst.

8. Applikatorsystem zur Anwendung bei der Strahlentherapie, umfassend:
- den Applikator (1) nach einem der vorhergehenden Ansprüche; und
- eine Mehrzahl von Absorptionskörpern (4), welche jeweils mit dem Grundkörper (2) des Applikators (1) verbindbar sind und welche jeweils die aus der Strahlungsquelle austretende Strahlung derart beeinflussen, dass eine bevorzugte Strahlungsrichtung und jeweils unterschiedliche Strahlcharakteristiken der austretenden Strahlung definiert sind.

## Claims

1. Applicator (1) for use in radiation therapy, comprising:
- a substantially cylindrically embodied main body (2) for holding a radiation source, wherein the main body (2) has a cavity extending along a main axis (A) of the main body in the interior thereof; and
- a substantially cylindrically embodied absorption body (4) which is movably connected to the main body (2) and which influences the radiation emerging from the radiation source in such a way that a preferred radiation direction is defined,
wherein the main body (2) and the absorption body (4) are arranged in succession in the longitudinal direction and the plane in which the ends of the two cylinders abut against one another is angled relative to the main axis (A) of the main body,
**characterized in that** the absorption body (4) is rotatably connected to the main body (2) and the axis of rotation (D) of the absorption body (4) is angled with respect to the main axis (A) of the main body such that the preferred radiation direction can be tilted into a plurality of different positions relative to the main axis (A) of the main body.

2. Applicator (1) according to Claim 1, **characterized in that** the absorption body (4) is detachably connected to the main body (2).

3. Applicator (1) according to either of Claims 1 and 2, **characterized in that** a locking means (6) is provided for locking a position of the absorption body (4) with respect to the main body (2).

4. Applicator (1) according to Claim 3, **characterized in that** the locking means comprises a retaining ring (6).

5. Applicator (1) according to either of Claims 3 and 4, **characterized in that** the absorption body (4) can be locked into a plurality of different positions relative to the main body (2), wherein the preferred radiation direction relative to the main axis (A) of the main body assumes a different value for each position.

6. Applicator (1) according to either of Claims 3 and 4, **characterized in that** the absorption body (4) can be moved continuously relative to the main body (2) and can be locked in any position by the locking means (6).

7. Applicator (1) according to one of Claims 1 to 6, **characterized in that** the absorption body (4) comprises an absorption means holder and an absorption means, wherein the absorption means is embodied in such a way that it influences beam characteristics, such as diameter, intensity and propagation direction, of the emerging radiation.

8. Applicator system for use in radiation therapy, comprising:
- the applicator (1) according to one of the preceding claims; and
- a plurality of absorption bodies (4), which can each be connected to the main body (2) of the applicator (1) and which each influence the radiation emerging from the radiation source in such a way that a preferred radiation direction and in each case different beam characteristics of the emerging radiation are defined.

## Revendications

1. Applicateur (1) destiné à être utilisé en radiothérapie, comprenant :
- un corps de base (2) réalisé de manière à présenter une forme sensiblement cylindrique destiné à recevoir une source de rayonnement, dans lequel le corps de base (2) présente à l'intérieur de celui-ci une cavité qui s'étend le long d'un axe principal du corps de base (A) ; et
- un corps d'absorption (4) réalisé de manière à présenter une forme sensiblement cylindrique, qui est relié de manière mobile au corps de base (2), et qui agit sur le rayonnement émanant de la source de rayonnement de manière à définir une direction de rayonnement préférée,
dans lequel le corps de base (2) et le corps d'absorption (4) sont disposés l'un derrière l'autre dans la direction longitudinale et le plan dans lequel les extrémités des deux cylindres sont en butée l'une contre l'autre est incliné par rapport à l'axe principal (A) du corps de base,
**caractérisé en ce que** le corps d'absorption (4) est relié tournant au corps de base (2) et l'axe de rotation (D) du corps d'absorption (4) est incliné par rapport à l'axe principal du corps de base (A), de telle manière que la direction de rayonnement préférée puisse être inclinée par rapport à l'axe principal du corps de base (A) dans une pluralité de positions différentes.

2. Applicateur (1) selon la revendication 1, **caractérisé en ce que** le corps d'absorption (4) est relié de manière amovible au corps de base (2).

3. Applicateur (1) selon l'une quelconque des revendications 1 ou 2, **caractérisé en ce qu'**il est prévu un moyen de blocage (6), afin de bloquer une position du corps d'absorption (4) par rapport au corps de base (2).

4. Applicateur (1) selon la revendication 3, **caractérisé en ce que** le moyen de blocage comprend une bague de sécurité (6).

5. Applicateur (1) selon l'une quelconque des revendications 3 ou 4, **caractérisé en ce que** le corps d'absorption (4) peut être bloqué dans une pluralité de positions différentes par rapport au corps de base (2), dans lequel la direction de rayonnement préférée prend une autre valeur par rapport à l'axe principal (A) du corps de base pour chaque position.

6. Applicateur (1) selon l'une quelconque des revendications 3 ou 4, **caractérisé en ce que** le corps d'absorption (4) est mobile en continu par rapport au corps de base (2) et peut être bloqué à chaque position quelconque par le moyen de blocage (6).

7. Applicateur (1) selon l'une quelconque des revendications 1 à 6, **caractérisé en ce que** le corps d'absorption (4) comprend un support de moyen d'absorption et un moyen d'absorption, dans lequel le moyen d'absorption est réalisé de manière à agir sur des caractéristiques de rayonnement, comme par exemple le diamètre, l'intensité et la direction de propagation du rayonnement sortant.

8. Système applicateur destiné à être utilisé en radiothérapie, comprenant :
- l'applicateur (1) selon l'une quelconque des revendications précédentes ; et
- une pluralité de corps d'absorption (4) qui peuvent respectivement être reliés au corps de base (2) de l'applicateur (1) et qui agissent respectivement sur le rayonnement émanant de la source de rayonnement de manière à définir une direction de rayonnement préférée et des caractéristiques de rayonnement respectives différentes du rayonnement émis.
